# EUROPEAN PATENT SPECIFICATION

(11) **EP 3 609 473 B1**
(45) Date of publication and mention of the grant of the patent: **10.01.2024**
(21) Application number: 18721156.0
(22) Date of filing: 09.04.2018
(51) Int. Cl.: A61K 9/08, A61K 33/26, A61K 9/28, A61P 7/06

(54) **ORAL COMPOSITIONS FOR THE TREATMENT OF IRON DEFICIENCY DISORDERS**
ORALE ZUSAMMENSETZUNGEN ZUR BEHANDLUNG VON EISENMANGELSTÖRUNGEN
COMPOSITIONS ORALES POUR LE TRAITEMENT DE TROUBLES DE CARENCE EN FER

(30) Priority: 10.04.2017 IT 201700039524
(43) Date of publication of application: 19.02.2020
(73) Proprietor: Labomar S.p.A., 31036 Istrana (TV) (IT)
(72) Inventor: FRATTER, Andrea, 31036 Istrana TV (IT); BERTIN, Walter, 31036 Istrana TV (IT)
(74) Representative: Perani & Partners S.p.A.
(86) International application number: PCT/IB2018/052456
(87) International publication number: WO 2018/189649

(56) References cited:
- WO-A1-01/12163
- WO-A1-2009/128795
- WO-A1-2016/041995
- "Fresh & Fruity Multivitamins", GNPD; MINTEL, 31 December 2012 (2012-12-31), XP002721234,
- A. H. MARTIN ET AL: "Enhancing the in vitro Fe2+ bio-accessibility using ascorbate and cold-set whey protein gel particles", DAIRY SCIENCE AND TECHNOLOGY, vol. 92, no. 2, 27 January 2012 (2012-01-27), pages 133-149, XP055170146, ISSN: 1958-5586, DOI: 10.1007/s13594-011-0055-0

## Description

### FIELD OF THE INVENTION

The present invention relates to an oral composition in solid or liquid form for the transport of bivalent iron in the form of a stable complex in aqueous medium at a pH of between 4.8 and 6.8.

### PRIOR ART

Iron deficiency is one of the most widespread nutritional disorders in the world. According to the World Health Organization, it is the only nutritional deficiency that not only afflicts developing countries, but also finds a high spread in industrialized countries. Iron deficiency notoriously results in sideropenic or martial anaemia, which globally affects more than 30% of the population. The categories most affected by iron deficiency anaemia are children, adolescents, childbearing-aged women and pregnant women.

Iron is an essential mineral, namely, it is an indispensable substance for survival, but cannot be synthesized by human metabolism. Its supply must therefore be guaranteed through food. In western countries, the bioavailability of iron taken from food does not exceed 15-20%. Iron is involved in various metabolic processes including: haemoglobin and myoglobin synthesis, which can bind and transport oxygen and release it in response to specific requests; DNA synthesis as a component of ribonucleotide reductase; electron transport in oxidative phosphorylation for the formation of ATP in mitochondria.

Iron occurs in two oxidative states, Fe²⁺ reduced iron and Fe³⁺ oxidized iron. In human organism, it is mainly bound to proteins in the form of non-EME iron or in the chelate form of EME iron. The EME group is a complex characterized by a metallic heart represented by an iron atom in the oxidation state Fe²⁺ and by a molecule of proto porphyrin IX.

More than 60% of the iron in the organism is found in the erythrocytes bound as EME iron to haemoglobin, where it reversibly binds an oxygen molecule and transports it from the lungs to the tissues. The reduced presence of iron in the organism causes the onset of martial anaemia, because the production of haemoglobin and the transport of oxygen are compromised.

Physiologically, the processes of absorption, use, transport and storage guarantee iron homeostasis. The elimination metabolism has no relevant influence, since only small amounts of iron, about 0.5-1 mg, are eliminated daily through processes of epithelial and gastrointestinal desquamation and through possible bleeding phenomena. The absorption process is therefore crucial to ensure the correct supply of iron and the biological activities in which it is involved. The onset of sideropenic anaemia is the result of an imbalance between absorption, use and storage. The iron stored in the form of ferritin and hemosiderin is progressively used without being compensated by the absorption process.

The alteration of iron homeostasis can be caused by: 1) low intake of iron in the diet, due to eating disorders or to an unbalanced diet; 2) inadequate absorption due to genetic disorders or pathologies; 3) increased need associated with pregnancy, lactation and periods of growth; 4) protracted loss due to bleeding, menstrual cycle or pathological infections. The prevailing symptoms are fatigue, weakness, paleness, chest pain and increased heart rate.

The iron taken with the diet is 90% in ionic form and 10% in the blood form. Non-EME iron is present in cereals, legumes, fruit and plants, while EME iron is assumed through meat and fish. Absorption occurs in the duodenum and proximal jejunum by enterocytes with different mechanisms for the two forms. The absorption of iron in non-EME form is mediated by the bivalent metal transporter 1 (DCT1), an H⁺/Fe²⁺ co-transporter, located on the enterocyte membrane. Iron is internalized in the form of ferrous iron and the acidification by the chyme of the first intestinal tract favours the uptake process (Jacobs A., Miles PM Role of gastric secretion in iron absorption.) Gut. 1969 Mar; 10(3): 226-229 PMCID: PMC1552819).

The absorption of the oxidized form Fe³⁺ occurs only after a reduction by means of duodenal cytochrome b (DCYTB). DCYTB is a ferric transmembrane reductase located in the apical portion of enterocytes, which uses cytoplasmic ascorbate as an electron donor for iron 3+ reduction (Oakhill JS et al. Functional characterization of human duodenal cytochrome b (Cybrd1): Redox properties in relation to iron and ascorbate metabolism, Biochim. Biophys. Acta, 2008 Mar; 1777 (3):260-8, doi: 10.1016/j.bbabio.2007.12.001 Epub 2007 Dec 23.) (Su D., Asard H. Three mammalian cytochromes b561 are ascorbate-dependent ferrireductases. FEBS J. 2006 Aug; 273 (16): 3722-34.). Although reductase ensures the reduction and subsequent uptake of Fe³⁺, the absorption of ferric iron is limited if compared to the internalization of ferrous iron due to the saturability of the system.

The absorption of iron in the form of salt in reduced form is in any case influenced by the tendency of ferrous iron to oxidise to ferric iron in the presence of atmospheric oxygen and oxygen present in gastric fluids.

The mechanism of absorption of EME iron is still a source of debate. Two hypotheses are mainly supported. The first one supports the existence of a mechanism of endocytosis able to internalize EME iron. The second one, instead, provides the involvement of membrane transporters for the transfer of the complex from the intestinal lumen into the enterocytes (West AR, Oates PS Mechanisms of heme iron absorption: Current questions and controversies, World J Gastroenterol, 2008 Jul 14; 14 (26): 4101-4110 Published online 2008 Jul 14. doi: 10.3748/wjg.14.4101 PMCID: PMC2725368).

Although the absorption mode of EME iron is not yet clear, it has been demonstrated that its bioavailability is higher and less influenced by other factors than the bioavailability of inorganic iron. Respectively, the absorption ranges are 15-35% for EME iron and 2-20% for non-EME iron.

The treatment of iron deficiency anaemia involves the integration of iron into the diet. Iron is administered in the form of salt, such as iron sulphate, iron fumarate, iron pyrophosphate in the form of tablets or drops. However, there are important side effects such as constipation, nausea and the onset of gastric lesions on an irritative basis, which in some cases compromise the continuation of treatment (Hashash JG Iron Pill-Induced Gastritis..ACG Case Rep J. 2013 Oct 8;1(1): 13-5.00 doi: 10.14309/ crj.2013.7. eCollection 2013.).

The intake of supplements or foods enriched with iron is further limited by the unpleasant taste of the active ingredient that lasts over time.

The problems of gastric damage and bad taste have recently been overcome with the integration of pyrophosphate iron in a lipo-sucrosomial form, in which a double phospholipidic layer protects the active ingredient. This technology allows the iron to be released only in the duodenal environment, thus bypassing the side effects produced in the stomach and the unpleasant taste.

"Fresh and Fruity multivitamins" GNPD; MINTEL, 31 December 2012 XP00273124 reports the components of the supplement marketed by Pfizer under the tradename Centrum which contains iron salts, vitamin E, vitamin C, gelatine (i.e. a peptide protein complex) and magnesium oxide. This supplement is used for its antioxidant properties and to strengthen the immune system.

WO01/12163 discloses a formulation for the treatment of anaemia that involves the presence of two distinct ferrous salts (a slow-dissolving one and a fast-dissolving one) and proteins, containing as optional components vitamin E and vitamin C, and in which the protein has only the function of binder in the powder granulation phase before the formation of the tablets.

A. H. Martin et al in "Enhancing the in vitro Fe2+ bioaccessibility using ascorbate and cold sets whey gel particles" DAIRY SCIENCE AND TECHNOLOGY, vol.92, no.2 pages 133-149, XP055170146 describe the formation of gels containing the salt complex of Fe(II) ascorbate proteinate by a laborious process including the following stages:
a) Preparation of protein solutions. The whey protein isolate (WPI) was dissolved in water at concentrations of 9% by weight and heated for 30 minutes at 80°C. Subsequently, this solution was cooled by diluting it to a final concentration of 6%. It was brought to pH 7 and stored cold.
b) Preparation of Fe(II) ascorbate solutions by mixing a 1 M solution of ferrous sulphate and an aqueous solution 1 M of sodium ascorbate, wherein the obtained solution is cold stored until further use.
c) Gel formation. The aqueous solution obtained in step b) is added to the solution of step a) and allowed gelling overnight at room temperature,
d) Formation of gel particles. The gel formed in step d) is turned into particles by pressing on a sieve, wash and lyophilisation.

WO2009/128795 describes liquid formulations containing iron in precise concentrations and vitamin C in precise concentrations, in which iron can be bivalent or trivalent and, only when it is trivalent, may be in the form of a protein-succinate compound.

WO2016/041995 describes microspheres having a protein matrix (preferably milk proteins) and incorporating the ferrous salt. These protein matrix microspheres are prepared by a procedure very similar to the one described by A.H. Martin et al in the aforementioned article.

Also this article and the aforesaid international application involve the possibility of using a protein such as whey protein and ascorbic acid, not only to mask the bad taste of ferrous salts but also because in this way the ferrous salt is less prone to oxidative stress thanks to the presence of the protein and is also mainly absorbed at the duodenal level, although both require a complex preparation procedure.

Therefore, there is a need to have Fe(II)-containing formulations:
- in which Fe(II) is easier to obtain,
- when the formulations come into contact with body fluids, they are less subject to oxidative stress and
- which can also be administered to people suffering from gastroduodenal disorders.

### SUMMARY OF THE INVENTION

The applicant has now found that the FeII-ascorbate-proteinate complex is formed in an aqueous medium containing vitamin E and magnesium oxide at a pH of between 4.8 and 6.8. This type of complex is preferably formed by means of the following two different methods:
(1) by dissolving in aqueous medium at the above pH range a solid oral composition (A) comprising an organic or inorganic ferrous salt, vitamin E, sodium ascorbate, plant or animal proteins and magnesium oxide in combination with suitable excipients and/or diluents such as e.g. fillers, or
(2) by forming a liquid composition (B) comprising the same components of the solid oral composition (A) and water at the aforesaid pH range.

The objects of the present invention are therefore a solid composition (A), for use in the treatment of disorders caused by iron deficiency, suitable to be dissolved in an aqueous medium according to the method (1); and a liquid composition (B), for use in the treatment of disorders caused by iron deficiency, prepared according to the method (2) for use in the treatment of disorders caused by iron deficiency.

When the solid oral composition (A) comes in contact with an aqueous medium having a pH of between 4.8 and 6.8, a Fe^{II}-ascorbate-proteinate complex forms in said aqueous medium. The liquid oral composition (B), since it comprises an aqueous medium having a pH between 4.8 and 6.8, contains a Fe^{II}-ascorbate-proteinate complex, as well as vitamin E and magnesium oxide. Said liquid composition B) is prepared extemporaneously or not extemporaneously by dispersing in water at pH between 4.8 and 6.8:
- an organic or inorganic ferrous salt, vitamin E, sodium ascorbate, plant or animal proteins and magnesium oxide or
- the oral solid composition (A) for the use as mentioned above.

The applicant has in fact surprisingly found that the solid oral composition (A), or the liquid oral composition (B), comprising the above mentioned compounds, which are able to combine with each other for conveying inorganic or organic ferrous iron in the form of the FeII-ascorbate-proteinate complex according to the present invention, can promote the absorption and maintenance of reduced iron and has good organoleptic characteristics.

The processes for preparing the aforesaid oral compositions (A) or (B), for use according to the present invention, are also disclosed in the present description.

In particular, the method of preparation of the solid composition (A) comprises the following steps:
a) the protein, sodium ascorbate and magnesium oxide are mixed, preferably after sieving, preferably in the presence of a filler,
b) vitamin E is separately added in small portions to the powdered ferrous salt,
c) the mixture obtained in step (b) is poured onto the solid mixture obtained in step (a) and the relative sieving is carried out,
d) the excipients known in the field are added to obtain a powder suitable for administration. The oral composition in liquid form (B) is extemporaneous or non-extemporaneous.

Preferably, the method for preparing the non-extemporaneous liquid formulation (B) comprises the following steps:
a') sodium ascorbate and protein are dissolved in water and then magnesium oxide is suspended,
b') a mixture of a powdered salt of FeII and vitamin E is separately prepared,
c') the mixture of step b') is poured into the aqueous suspension obtained in step a'),
d') the excipients known in the field are added to obtain a solution suitable for oral administration.

Preferably, the process for the extemporaneous liquid formulation (B) involves the aqueous reconstitution of the solid oral composition (A) prepared by means of the aforesaid process.

### DESCRIPTION OF THE FIGURES

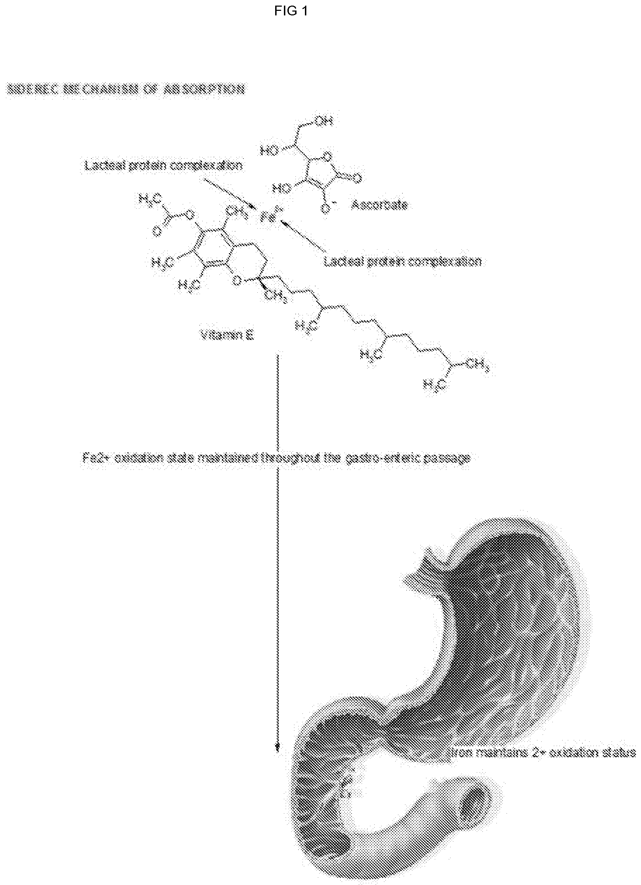

Figure 1 shows the formation scheme of the Fe^{II}-ascorbate-proteinate complex according to the present invention, in which, in particular, the protein is at least one whey protein and the bivalent iron salt is the bisglycinate iron, and its mechanism of action inside the stomach.

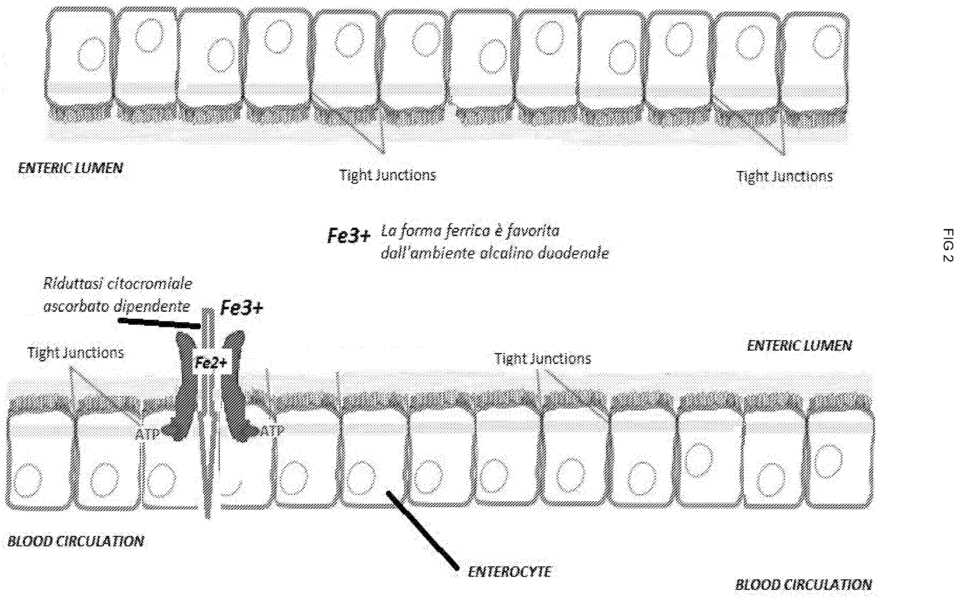

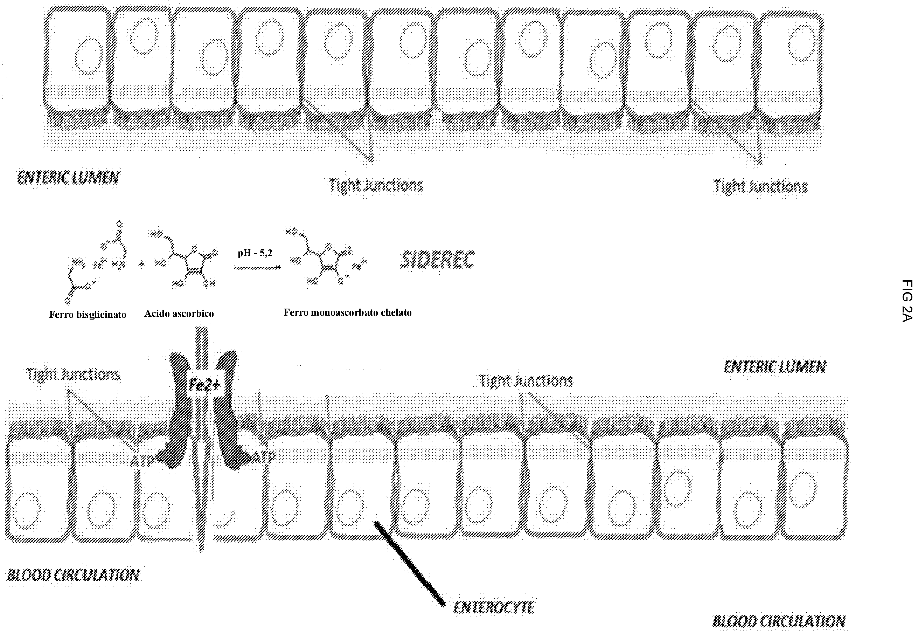

Figures 2 and 2A show the mechanism of absorption of the Fe ion in the intestine with or without chelation with FeII ascorbate and protein, clearly demonstrating that the oxidation of Fe^{II} to Fe^{III} is avoided only with the formation of the complex of the invention. The word Siderec in Figure 2A is the name given to the complex of the invention.

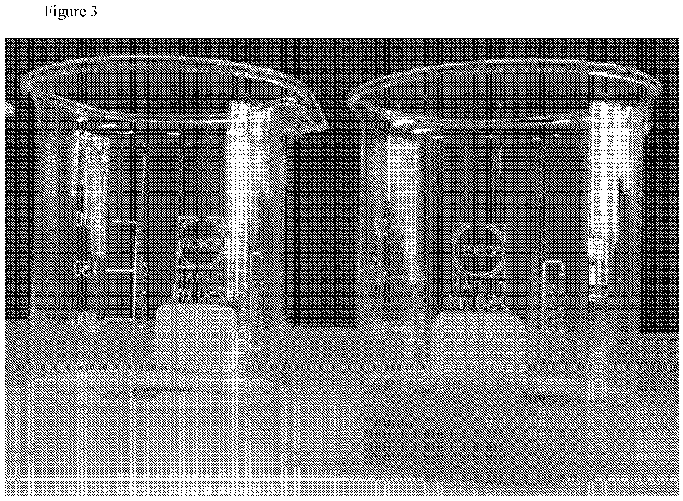

Figure 3 shows the photo of the result of an experiment, conducted first in a simulated gastric acid environment at pH 1.5 and then in an intestinal environment at pH 6.0, with the formulation (A) for use according to the present invention and compared to the same experiment using only bisglycinate iron.

### DETAILED DESCRIPTION OF THE INVENTION

For the purposes of the present invention, the definition "aqueous medium" refers to water already present in the liquid composition (B) and/or body fluids such as saliva, gastric juices, intestinal fluids in which the solid composition (A) or the liquid composition (B) comes into contact during and after the relative administration.

As previously stated, the oral compositions allowing the obtainment of the aqueous medium according to the present invention containing the Fe^{II}-ascorbate-proteinate complex are the solid composition (A) or the liquid composition (B).

For the purposes of the present invention, solid composition (A) is preferably in the form of an orodispersible powder or a water-reconstitutable powder, a compressible powder or a powder for filling rigid capsules.

The definition compressible powder means a solid formulation for generating tablets with a hardness of not less than 7 kg.

For the purposes of the present invention, powder for filling hard capsules means a powder having adequate flow characteristics and chemical-physical parameters to be processed in machines for filling hard capsules.

As mentioned above, the liquid oral composition (B) can be extemporaneous or non-extemporaneous.

For the purposes of the present invention, non-extemporaneous liquid oral composition (B) means a liquid formulation prepared and sold in this form. The extemporaneous liquid oral composition (B) is prepared on the spot by dispersion of the solid composition (A).

For the purposes of the present invention, the term Fe^{II}-ascorbate-proteinate means the proteinate chelate mono-ascorbate iron complex, whose schematic formula is shown in Figure 1.

The characteristic chemical mechanism of formation of the aforesaid complex from the solid formulation (A) or the liquid formulation (B) involves the formation in aqueous environment of the Fe²⁺-ascorbate monomeric chelating complex and the concomitant formation of the complex mediated by ionic interactions with the whey proteins (Fig 1,2). The chelate complex is monodentate and the metal is coordinated through the deprotonated C3 enolic function.

It has surprisingly been found that the formation of the Fe^{II}-ascorbate-proteinate complex allows the obtainment of a chelate compound which, assisted by its being bound with the proteins, during the gastric transit keeps the iron in the reduced state, which is the form preferably absorbed by the first tract of the small intestine.

The bivalent iron salt used in the preparation of the complex object of the present invention can be a pharmaceutically acceptable inorganic salt. It preferably is ferrous sulphate or an organic salt such as gluconate, lactate, fumarate, and all ferrous salts with amino acids where ferrous bisglycinate is particularly preferred.

In the solid formulation (A) or in the liquid formulation (B) object of the present invention, the formation of the complex according to the present invention is determined by a precise pH range.

In fact, it is essential that the aqueous environment of the composition (A) according to the present invention or of the liquid formulation (B) has a pH ranging from 4.8 to 6.8.

To ensure the correct pH range of the formulation (A) or of the formulation (B), said compositions are preferably further added with an acid, more preferably ascorbic acid.

In the above composition (A) or (B), the appropriate pH range in the gastric lumen is guaranteed by the presence of magnesium oxide. Once the formulation, both in solid form and in liquid form, reaches the stomach, magnesium oxide ensures a slight increase in gastric pH so as to settle it in the range necessary for the formation and maintenance of the complex. The protein used to prepare the complex according to the present invention is preferably an animal protein, such as e.g. at least a whey protein.

Whey proteins are globular proteins including albumins, lactoglobulins and immunoglobulins present in whey after the cheese formation process.

Physiologically present in the blood, albumins and lactoglobulins are responsible for the transport of other molecules, whereas immunoglobulins are involved in the processes of innate immunity. In nutraceutics, they are used as a source of proteins and essential amino acids mainly for muscle trophism and for the energy production through glucose synthesis. Other types of animal proteins that can be used in the preparation of the complex object of the present invention are caseinates of alkaline metals or of alkaline earth metals such as sodium or calcium caseinate.

As far as plant proteins are concerned, these are preferably soy and pea proteins.

As reported by Plug et al. and Muneta et Kaisakila, the formation of the ferrous iron-ascorbate complex generates a purple colour of the solution, which confirms the presence of the complexed reduced iron. The ferric iron-ascorbate chelating complex generates a light-yellow solution (Plug CM, Dekker D., Bult A. Complex Stability of ferrous ascorbate in aqueous solution and its significance for iron absorption.) Pharm Weekbl Sci. 1984 Dec 14; 6 (6): 245-8.) (Muneta P., Kaisaki F, Ascorbic acid-iron) iron (Fe++) complexes and after cooking darkening of potatoes Potato Journal (1985) 62: 531. doi: 10.1007/BF02854400)

In the solid composition (A) or in the liquid composition (B) object of the present invention, the maintenance of the reduced iron form is assisted by the presence in the formulation of vitamin E, which, during the phase of said compositions, is the first to be put in contact with the bivalent iron salt, thus creating a water-refractory environment, and then an oxygen-refractory environment, trying to further preserve the oxidative state of the iron before the actual formation of the complex.

The following comparative experiment is carried out to demonstrate the efficacy of the solid compositions (A) or of the liquid formulations (B). The results are shown in Figure 3.

The solid composition (A) is maintained for 90 minutes (corresponding to the empty stomach emptying time) in a simulated gastric environment (SGF) pH 1.5 by adding HCl, thermostated at 37°C. Initially, the system has a light purple colour, which indicates the formation of the ascorbate-proteinate-ferrous iron complex. Over time, the reduction of pH to values below 4.8 causes a clearing of the dispersion until a whitish liquid is obtained.

Subsequently, 50 ml of simulated intestinal fluid (SIF) are added to the system to simulate the transit in the first intestinal tract and the pH is settled at 6.0 with NaOH 2.5% w/v. The solution takes a more intense purple colour and demonstrates the presence of iron in reduced form. On the contrary, the formulation with only bisglycinate iron in SIF produces an amber-coloured liquid, which indicates the formation of no more reducible ferric oxides.

In the oral composition in solid form (A) or in liquid form (B), preferably the stoichiometric ratio of iron: sodium ascorbate is 1:1.

The remaining positive charge of the bivalent iron of the complex contained in the aqueous medium according to the present invention is neutralized by one of the two negative charges present in two molecules or in a single molecule of the counter-ion of the ferrous salt present in the solid oral composition (A) or in the liquid oral composition (B). This means that if the ferrous salt is ferrous sulphate, the remaining positive charge will be one of those present in the same sulphate anion or other anion present in the system. The same happens when the counter-ion is the fumarate. If the salt is glycinate or ferrous gluconate, the remaining positive charge of the complex formed according to the present invention will be neutralized by the only negative charge present in only one of the two anions forming with the divalent iron the ferrous bisglycinate or the ferrous bisgluconate, or by another anion present in the system.

For the purposes of the present invention, the solid composition (A) or the liquid composition (B) preferably has an animal or plant protein content of between 0.1% and 10% by weight out of the total weight of said composition (A) or (B).

For the purposes of the present invention, the solid composition (A) or the liquid formulation (B) preferably has a content of MgO comprised between 0.01% and 15% by weight out of the total weight of the composition (A) or (B).

For the purposes of the present invention, the vitamin E content is preferably between 1% and 15% by weight based on the total weight of the composition (A) or the composition (B). In the process according to the present invention, preferably in step a) of the process according to the present invention, polyols (e.g., mannitol, sorbitol, inositol, isomalt and xylitol, sugars such as e.g. sucrose, fructose and dextrose, calcium phosphate, starch and relative mixtures of said fillers) are used as filler.

Both in step b) of the formation process of the solid composition (A) or of the extemporaneous liquid composition (B) and in step b') of the preparation process of the non-extemporaneous liquid formulation (B), a hydrophilic emulsifier, and more preferably polysorbate 80, sucresters or mixtures thereof, is preferably added.

In particular, the solid oral composition (A) or the liquid oral composition (B) of the present invention is intended for the treatment of sideropenic anaemia. The formulation object of the present invention allows creating a Fe^{II}-ascorbate-proteinate complex to keep the iron in a reduced state, protecting it from oxidation phenomena and favouring its consequent absorption.

The presence of iron in a complexed form also guarantees a reduction of the side effects associated with the classic administration of iron in the form of salt.

In combination with these effects of increasing the iron bioavailability and of improving the taste pleasantness, the presence of MgO makes the compositions object of the present invention preferably, but not exclusively for subjects suffering from gastritis, and the concomitant presence of vitamin E and vitamin C promotes skin-restoring effects and prevention effects against relapses caused by Helicobacter pylori.

The iron integration in individuals with gastritis has always been a limiting factor due to the gastro-damaging action generated by the active ingredient, which would worsen the already present inflammatory state. The assumption of the compositions in question not only allows reducing all undesirable effects due to iron, but promotes an increase in gastric pH, thus limiting the damaging action of hydrochloric acid.

The causes that determine the onset of sideropenic anaemia include diseases associated with the gastrointestinal tract such as gastritis, peptic ulcer disease, stomach cancer and Helicobacter pylori infections as they cause bleeding and haemorrhage that alter iron homeostasis (Annibale B. et al. Gastrointestinal causes of refractory iron deficiency anaemia in patients without gastrointestinal symptoms Am J Med. 2001 Oct 15; 111 (6): 439-45.). The literature reports that both vitamin E and vitamin C have protective effect against rat injured gastric mucosa (Ohta Y et al. Vitamin E protects against stress-induced gastric mucosal lesions in rats more effectively than vitamin C. Biofactors 2010 Jan-Feb; 36 (1): 60-9.00: 10.1002/biof.73) and their co-administration promotes the eradication of Helicobacter pylori (Sezikli M.et to Oxidative stress in Helicobacter pylori infection: does supplementation with C and E increase the eradication rate? Helicobacter 2009 Aug; 14 (4): 280-5.00: 10.1111/j.1523-5378.2009.00686.x.).

### EXAMPLES

The following examples of the solid oral composition (A) or of the liquid oral composition (B) object of the present invention are reported for purely illustrative and non-limiting purposes.

### EXAMPLE 1

Table 1 shows the concentrations, by weight/weight, of the components making up a powder constituted by the formulation object of the present invention, in the form of a porous powder, or to be reconstituted in 100 ml of water.

| **Component** | **Concentration weight/total weight composition** |
|---|---|
| Bisglycinate chelate iron (iron 20%) | 7.50% |
| Liquid Vitamin E acetate (Alpha Tocopherol Equivalent 67%) | 5.82% |
| Polysorbate 80 | 3.75% |
| Whey proteins | 2.50% |
| Sodium ascorbate | 5.32% |
| Vitamin C | 6.90% |
| Sorbitol | 19.00% |
| Mannitol | 30.24% |
| Magnesium Oxide | 2.50% |
| Precipitated amorphous silica | 3.15% |
| Amorphous silica Sipernat 2200 | 1000% |
| Sucralose | 0.07% |
| Natural lemon flavouring powder | 1.75% |
| Strawberry flavouring powder | 1.50% |

The pharmaceutical powder is obtained by the process described below.
1. Mixing, after sieving, mannitol and sorbitol with whey proteins, sodium ascorbate, ascorbic acid and magnesium oxide.
2. Separately moistening the bisglycinate chelate iron with vitamin E acetate in the form of 67% oil.
3. Adding polysorbate 80 to the mixture of step 2.
4. Gradually pouring under stirring the mixture of step 3 into the mixture of powders of step 1.
5. Adding, after sieving, the silica to the mixture of step 4.
6. Sifting several times the whole mixture of step 5.
7. Adding sucralose, natural lemon flavouring powder and strawberry flavouring powder to the mixture of step 6.

### EXAMPLE 2

Table 2 shows the concentrations, by weight/weight, of the components making up a powder constituted by the formulation object of the present invention, in the form of a tablet.

| Component | Concentration weight/total weight composition |
|---|---|
| Bisglycinate chelate iron (iron 20%) | 25.00% |
| Liquid vitamin E acetate (Alpha Tocopherol Equivalent 67%) | 2.99% |
| Whey proteins | 4.17 % |
| Sodium ascorbate | 17.73% |
| Sucrester | 3.88% |
| Magnesium Oxide | 8.33% |
| Pregelatinised corn starch | 27.90% |
| Dibasic calcium phosphate dihydrate | 8.33% |
| Precipitated amorphous silica | 0.83% |
| Magnesium Stearate | 0.83% |

The pharmaceutical powder is obtained by the process described below.
1. Mixing, after sifting, sodium ascorbate, whey protein, sucrester, magnesium oxide, pregelatinised corn starch, dibasic calcium phosphate dihydrate.
2. Separately moistening the bisglycinate chelate iron with vitamin E acetate in the form of 67% oil.
3. Gradually pouring under stirring the mixture of step 2 into the mixture of powders of step 1.
4. Adding precipitated amorphous silica and magnesium stearate to the mixture of step 3.
5. Sifting the mixture of step 4.
6. Compressing the mixture of step 5.
7. Filming the tablets of step 6 with the aid of commonly used excipients for the production of a gastroresistant film.

### EXAMPLE 3

Table 3 shows the concentrations, by weight/weight, of the components making up a powder constituted by the formulation object of the present invention, in the form of a capsule.

| **Component** | **Concentration weight/total weight composition** |
|---|---|
| Bisglycinate chelate iron (iron 20%) | 31.25% |
| Liquid vitamin E acetate (Alpha Tocopherol Equivalent 67%) | 3.73% |
| Whey proteins | 5.21% |
| Sodium ascorbate | 22.17% |
| Sucroester | 4.85% |
| Magnesium Oxide | 10.42% |
| Pregelatinised corn starch | 1988% |
| Precipitated amorphous silica | 1.25% |
| Magnesium Stearate | 1.25% |

The pharmaceutical powder is obtained by the process described below.
1. Mixing, after sifting, sodium ascorbate, whey protein, sucrester, magnesium oxide and pregelatinised corn starch.
2. Moistening the bisglycinate chelate iron with vitamin E acetate in the form of 67% oil.
3. Gradually pouring under stirring the mixture of step 2 into the mixture of powders of step 1.
4. Adding precipitated amorphous silica and magnesium stearate to the mixture of step 3.
5. Sifting the mixture of step 4.
6. Encapsulating in an operculum the mixture of step 5.

### EXAMPLE 4

Table 4 shows the concentrations, by weight/weight, of the components making up a solution in the form of drops constituted by the formulation object of the present invention.

| **Component** | **Concentration weight/total weight composition** |
|---|---|
| Bisglycinate chelate iron (20%) | 8.75% |
| Liquid vitamin E acetate (Alpha Tocopherol Equivalent 67%) | 9.70% |
| Polysorbate | 5.00% |
| Whey proteins | 4.50% |
| Sodium ascorbate | 6.25% |
| Magnesium Oxide | 0.10% |
| Vitamin C | 1000% |
| Wildflower honey | 1500% |
| Purified water | 40.50% |
| Potassium sorbate | 0.10% |
| Sodium benzoate | 0.10% |

The aqueous solution is obtained by the process described below.
1. Moistening the bisglycinate iron with the vitamin E acetate in the form of 67% oil.
2. Adding the polysorbate 80 to the mixture of step 1.
3. Adding honey to the mixture of step 2.
4. Separately dissolving sodium ascorbate, ascorbic acid and whey proteins in water.
5. Gradually pouring under stirring the mixture of step 3 into the solution of step 4.
6. Adding potassium sorbate and sodium benzoate to the system of step 5.

## Claims

1. Solid oral composition (A) comprising an organic or inorganic ferrous salt, vitamin E, sodium ascorbate, animal or plant proteins and magnesium oxide, in combination with suitable excipients and/or diluents for use in the treatment of disorders caused by iron deficiency, wherein, when said solid oral composition comes in contact with an aqueous medium having a pH of between 4.8 and 6.8 a Fe^{II}-ascorbate-proteinate complex forms in said aqueous medium containing also vitamin E and magnesium oxide.

2. Solid oral composition (A) for use according to claim 1, in the form of a water-dispersible powder, in the form of tablets, in the form of hard capsules, or in the form of orodispersible tablets.

3. Liquid oral composition (B) for use in the treatment of disorders caused by iron deficiency, comprising an aqueous medium having a pH between 4.8 and 6.8 containing Fe^{II}-ascorbate-proteinate complex, vitamin E and magnesium oxide, said liquid composition B) being prepared extemporaneously or not extemporaneously by dispersing in water at pH between 4.8 and 6.8:
• an organic or inorganic ferrous salt, vitamin E, sodium ascorbate, plant or animal proteins and magnesium oxide or
• the oral solid composition (A) for the use according to claim 1 or 2.

4. Solid oral composition (A) for use according to any of the claims 1, or liquid oral composition (B) for use according to claim 3, wherein said disorder caused by iron deficiency is sideropenic or martial anaemia.

5. Solid oral composition (A) for use according to any of the claims 1 or 2 , or liquid oral composition (B) for use according to claim 3, wherein said compositions have a high gastric tolerability and reduced risk of appearance of gastralgia, typically associated with iron supplementation.

6. Solid oral composition (A) or liquid oral composition (B) for use according to claim 4, wherein, sideropenic anaemia is associated with gastric disorders and lesions in patients also suffering from gastrointestinal diseases.

7. Solid oral composition (A) or liquid oral composition (B) for use according to claim 6, wherein said patients suffer from gastritis and/or gastric and duodenal ulcers in the presence of Helicobacter pylori colonization.

8. Solid oral composition (A) for use according to anyone of claims 1,2,4-7 or liquid oral composition (B) for use according any one of claim 3-7, wherein said organic or inorganic ferrous salt is selected among ferrous sulphate, ferrous gluconate, ferrous lactate, ferrous fumarate, ferrous salt with an amino acid, and preferably is ferrous bisglycinate.

9. Solid oral composition (A) for use according to any of the claims 1, 2, 4-8 , or liquid oral composition (B) for use according to any of the claims 3-8, wherein said animal or plant protein is chosen among at least one whey protein, a preferably sodium or calcium alkali metal or alkaline earth metal caseinate, soy and pea proteins.

10. Solid oral composition (A) for use according to any of the claims 1, 2 and 4-9, or liquid oral composition (B) for use according to any of the claims 3-9, wherein the stoichiometric ratio of iron:sodium ascorbate is 1:1.

11. Solid oral composition (A) for use according to any of the claims 1,2 and 4-10, or liquid oral composition (B) for use according to any of the claims 3-10, containing said animal or plant protein in amounts ranging from 0.1% to 10% by weight out of the total weight of said composition (A) or (B).

12. Solid oral composition (A) for use according to any of the claims 1, 2 and 4-11 or liquid oral composition (B) for use according to any of the claims 3-11, comprising magnesium oxide in amounts ranging from 0.01% to 15% by weight out of the total weight of the composition (A) or (B).

13. Solid oral composition (A) for use according to any of the claims 1,2,4-12 and liquid oral composition (B) for use according to any of the claims 4-12, comprising vitamin E in amounts ranging from 1% to 15% by weight out of the total weight of the composition (A) or (B).

## Patentansprüche

1. Feste orale Zusammensetzung (A), umfassend ein organisches oder anorganisches Eisensalz, Vitamin E, Natriumascorbat, tierische oder pflanzliche Proteine und Magnesiumoxid in Kombination mit geeigneten Hilfsstoffen und/oder Verdünnungsmitteln zur Verwendung bei der Behandlung von Störungen, die durch Eisenmangel verursacht werden, wobei, wenn die feste orale Zusammensetzung mit einem wässrigen Medium mit einem pH zwischen 4,8 und 6,8 in Kontakt kommt, ein Fe^{II}-Ascorbat-Proteinat-Komplex in dem wässrigen Medium gebildet wird, der auch Vitamin E und Magnesiumoxid enthält.

2. Feste orale Zusammensetzung (A) zur Verwendung nach Anspruch 1, in Form eines wasserdispergierbaren Pulvers, in Form von Tabletten, in Form von Hartkapseln oder in Form von Schmelztabletten.

3. Flüssige orale Zusammensetzung (B) zur Verwendung bei der Behandlung von Störungen, die durch Eisenmangel verursacht werden, umfassend ein wässriges Medium, das einen pH zwischen 4,8 und 6,8 aufweist und Fe^{II}-Ascorbat-Proteinat-Komplex, Vitamin E und Magnesiumoxid enthält, wobei die flüssige Zusammensetzung B) durch Dispergieren von Folgendem in Wasser bei einem pH zwischen 4,8 und 6,8 vorübergehend oder nicht vorübergehend hergestellt wird:
• einem organischen oder anorganischen Eisensalz, Vitamin E, Natriumascorbat, pflanzlichen oder tierischen Proteinen und Magnesiumoxid oder
• der oralen festen Zusammensetzung (A) zur Verwendung nach Anspruch 1 oder 2.

4. Feste orale Zusammensetzung (A) zur Verwendung nach einem der Ansprüche 1 oder flüssige orale Zusammensetzung (B) zur Verwendung nach Anspruch 3, wobei die Störung, die durch Eisenmangel verursacht wird, sideropenische oder martiale Anämie ist.

5. Feste orale Zusammensetzung (A) zur Verwendung nach einem der Ansprüche 1 oder 2 oder flüssige orale Zusammensetzung (B) zur Verwendung nach Anspruch 3, wobei die Zusammensetzungen eine hohe Magenverträglichkeit und ein verringertes Risiko eines Auftretens von Gastralgie aufweisen, das normalerweise mit einer Eisensupplementierung assoziiert ist.

6. Feste orale Zusammensetzung (A) oder flüssige orale Zusammensetzung (B) zur Verwendung nach Anspruch 4, wobei sideropenische Anämie mit gastrischen Störungen und Läsionen bei Patienten, die auch an gastrointestinalen Erkrankungen leiden, assoziiert ist.

7. Feste orale Zusammensetzung (A) oder flüssige orale Zusammensetzung (B) zur Verwendung nach Anspruch 6, wobei die Patienten an Gastritis und/oder Magen- und Zwölffingerdarmgeschwüren in Anwesenheit einer Kolonisation von Helicobacter pylori leiden.

8. Feste orale Zusammensetzung (A) zur Verwendung nach einem der Ansprüche 1, 2, 4-7 oder flüssige orale Zusammensetzung (B) zur Verwendung nach einem der Ansprüche 3-7, wobei das organische oder anorganische Eisensalz ausgewählt ist aus Eisensulfat, Eisengluconat, Eisenlactat, Eisenfumarat, Eisensalz mit einer Aminosäure und vorzugsweise Eisenbisglycinat ist.

9. Feste orale Zusammensetzung (A) zur Verwendung nach einem der Ansprüche 1, 2, 4-8 oder flüssige orale Zusammensetzung (B) zur Verwendung nach einem der Ansprüche 3-8, wobei das tierische oder pflanzliche Protein ausgewählt ist aus mindestens einem Molkenprotein, einem vorzugsweise Natrium- oder Calciumalkalimetall- oder Erdalkalimetallkaseinat, Soja- und Erbsenproteinen.

10. Feste orale Zusammensetzung (A) zur Verwendung nach einem der Ansprüche 1, 2 und 4-9 oder flüssige orale Zusammensetzung (B) zur Verwendung nach einem der Ansprüche 3-9, wobei das stöchiometrische Verhältnis von Eisen:Natriumascorbat 1:1 ist.

11. Feste orale Zusammensetzung (A) zur Verwendung nach einem der Ansprüche 1, 2 und 4-10 oder flüssige orale Zusammensetzung (B) zur Verwendung nach einem der Ansprüche 3-10, die das tierische oder pflanzliche Protein in Mengen im Bereich von 0,1 bis 10 Gewichtsprozent des Gesamtgewichts der Zusammensetzung (A) oder (B) enthält.

12. Feste orale Zusammensetzung (A) zur Verwendung nach einem der Ansprüche 1, 2 und 4-11 oder flüssige orale Zusammensetzung (B) zur Verwendung nach einem der Ansprüche 3-11, umfassend Magnesiumoxid in Mengen in einem Bereich von 0,01 bis 15 Gewichtsprozent des Gesamtgewichts der Zusammensetzung (A) oder (B).

13. Feste orale Zusammensetzung (A) zur Verwendung nach einem der Ansprüche 1, 2, 4-12 und flüssige orale Zusammensetzung (B) zur Verwendung nach einem der Ansprüche 4-12, umfassend Vitamin E in Mengen in einem Bereich von 1 bis 15 Gewichtsprozent des Gesamtgewichts der Zusammensetzung (A) oder (B).

## Revendications

1. Composition orale solide (A) comprenant un sel ferreux organique ou inorganique, de la vitamine E, de l'ascorbate de sodium, des protéines animales ou végétales et de l'oxyde de magnésium, en combinaison avec des excipients et/ou des diluants appropriés pour utilisation dans le traitement de troubles causés par une carence en fer, dans laquelle, lorsque ladite composition orale solide entre en contact avec un milieu aqueux présentant un pH compris entre 4,8 et 6,8, un complexe Fe^{II}-ascorbate-protéinate se forme dans ledit milieu aqueux contenant également de la vitamine E et de l'oxyde de magnésium.

2. Composition orale solide (A) pour utilisation selon la revendication 1, sous forme de poudre dispersible dans l'eau, sous forme de comprimés, sous forme de gélules, ou sous forme de comprimés orodispersibles.

3. Composition orale liquide (B) pour utilisation dans le traitement de troubles causés par une carence en fer, comprenant un milieu aqueux présentant un pH compris entre 4,8 et 6,8 contenant un complexe Fe^{II}-ascorbate-protéinate, de la vitamine E et de l'oxyde de magnésium, ladite composition liquide B) étant préparée extemporanément ou non extemporanément par dispersion dans de l'eau à un pH compris entre 4,8 et 6,8 :
• d'un sel ferreux organique ou inorganique, de la vitamine E, de l'ascorbate de sodium, des protéines végétales ou animales et de l'oxyde de magnésium ou
• de la composition solide orale (A) pour l'utilisation selon la revendication 1 ou 2.

4. Composition orale solide (A) pour utilisation selon l'une quelconque des revendications 1, ou composition orale liquide (B) pour utilisation selon la revendication 3, dans laquelle ledit trouble causé par une carence en fer est une anémie sidéropénique ou martiale.

5. Composition orale solide (A) pour utilisation selon l'une quelconque des revendications 1 ou 2, ou composition orale liquide (B) pour utilisation selon la revendication 3, dans laquelle lesdites compositions présentent une tolérance gastrique élevée et un risque réduit d'apparition de gastralgie, généralement associée à une supplémentation en fer.

6. Composition orale solide (A) ou composition orale liquide (B) pour utilisation selon la revendication 4, dans laquelle l'anémie sidéropénique est associée à des troubles et des lésions gastriques chez des patients souffrant également de maladies gastro-intestinales.

7. Composition orale solide (A) ou composition orale liquide (B) pour utilisation selon la revendication 6, dans laquelle lesdits patients souffrent de gastrite et/ou d'ulcères gastriques et duodénaux en présence d'une colonisation par Helicobacter pylori.

8. Composition orale solide (A) pour utilisation selon l'une quelconque des revendications 1, 2, 4-7 ou composition orale liquide (B) pour utilisation selon l'une quelconque des revendications 3-7, dans laquelle ledit sel ferreux organique ou inorganique est sélectionné parmi le sulfate ferreux, le gluconate ferreux, le lactate ferreux, le fumarate ferreux, le sel ferreux avec un acide aminé, et est de préférence le bisglycinate ferreux.

9. Composition orale solide (A) pour utilisation selon l'une quelconque des revendications 1, 2, 4-8, ou composition orale liquide (B) pour utilisation selon l'une quelconque des revendications 3-8, dans laquelle ladite protéine animale ou végétale est choisie parmi au moins une protéine de lactosérum, un caséinate de métal alcalin ou de métal alcalino-terreux de sodium, de préférence, ou de calcium, des protéines de soja et de pois.

10. Composition orale solide (A) pour utilisation selon l'une quelconque des revendications 1, 2 et 4-9, ou composition orale liquide (B) pour utilisation selon l'une quelconque des revendications 3-9, dans laquelle le rapport stoechiométrique fer:ascorbate de sodium est de 1:1.

11. Composition orale solide (A) pour utilisation selon l'une quelconque des revendications 1, 2 et 4-10, ou composition orale liquide (B) pour utilisation selon l'une quelconque des revendications 3-10, contenant ladite protéine animale ou végétale en des quantités allant de 0,1 % à 10 % en poids sur le poids total de ladite composition (A) ou (B).

12. Composition orale solide (A) pour utilisation selon l'une quelconque des revendications 1, 2 et 4-11 ou composition orale liquide (B) pour utilisation selon l'une quelconque des revendications 3-11, comprenant de l'oxyde de magnésium en des quantités allant de 0,01 % à 15 % en poids sur le poids total de la composition (A) ou (B).

13. Composition orale solide (A) pour utilisation selon l'une quelconque des revendications 1, 2, 4-12 et composition orale liquide (B) pour utilisation selon l'une quelconque des revendications 4-12, comprenant de la vitamine E en des quantités allant de 1 % à 15 % en poids sur le poids total de la composition (A) ou (B).
